# EUROPEAN PATENT APPLICATION

(11) **EP 4 803 110 A1**
(43) Date of publication of application: **09.09.2026**
(21) Application number: 25204630.5
(22) Date of filing: 25.09.2025
(51) Int. Cl.: A61L 27/46, A61L 27/50, A61L 27/52

(54) **RECOMBINANT COLLAGEN FILLER, PREPARATION METHOD THEREFOR AND USE THEREOF**

(30) Priority: 03.03.2025 CN 202510246962
(71) Applicant: WitKang Zhiyuan Medical Devices (Xi'an) Co., Ltd., Xi'an City Shaanxi 710075 (CN)
(72) Inventor: LI, Yanting, Xi'an City, Shaanxi Province, 710075 (CN); WANG, Meihua, Xi'an City, Shaanxi Province, 710075 (CN); CHEN, Liang, Xi'an City, Shaanxi Province, 710075 (CN); WANG, Jiuna, Xi'an City, Shaanxi Province, 710075 (CN); HOU, Jianing, Xi'an City, Shaanxi Province, 710075 (CN); WANG, Ya, Xi'an City, Shaanxi Province, 710075 (CN); LI, Yuan, Xi'an City, Shaanxi Province, 710075 (CN)
(74) Representative: IP TRUST SERVICES

(57) **Abstract**

The present disclosure provides a recombinant collagen filler, a preparation therefor, and the use thereof. The components of the recombinant collagen filler comprise a recombinant collagen gel and coral hydroxyapatite particles. The mass ratio of the recombinant collagen gel to the coral hydroxyapatite particles is (2.1-11) : 1. The filler further optionally comprises a first diluent. The content of the first diluent is 32.5-45.5% based on 100% of the total mass of the recombinant collagen filler. The coral hydroxyapatite particles are uniformly distributed in the recombinant collagen gel. The recombinant collagen gel is formulated from recombinant collagen microspheres and a second diluent. The filler of the present disclosure has a low cost and a high performance, the injection process thereof is fast and convenient, the action thereof is long-lasting, without a need for multiple injections, and the wrinkle removal effect thereof is long-acting and durable.

## Description

### TECHNICAL FIELD

The present disclosure belongs to the field of biomedical materials, relates to the field of injections for medical aesthetic filling, and specifically relates to a recombinant collagen filler, a preparation method therefor, and the use thereof.

### BACKGROUND

With the improvement of living standards, the demand of human beings for beauty is increasing day by day. As a safe and effective way, injectable fillers are increasingly favored by consumers. The injection filling technique is simple to operate, takes effect immediately after only a few minutes of injection, causes no pain, no interference with work or daily life, and has high convenience, no trace left, and good confidentiality, thereby protecting the privacy of customers. However, an ideal injectable filling material for soft tissue should have biocompatibility, safety, easy operation, anchoring property, and durability simultaneously, which has been a highly challenging topic for researchers who are engaged in the research and development of plastic surgery and medical aesthetic products.

At present, injectable minimally invasive aesthetic injection materials include hyaluronic acid, botulinum toxin, regenerative injectables, collagen, and other materials, which mainly work for filling plasticity, so as to eliminate facial wrinkles, achieve aesthetic appearance, and resist aging.

At present, commonly used injectable minimally invasive aesthetic filling materials in the world are as follows:
1. Hyaluronic acid fillers: Hyaluronic acid fillers are applicable to the whole face. Medium-to-high molecular weight hyaluronic acids have strong support capacity, and are suitable for filling and contouring, and there is basically no recovery period after injection. In addition, hyaluronic acid can be completely absorbed and is thus also a good choice in sites such as the top of the skull and the back of the head. However, the maintenance time is relatively short, and supplementation at regular intervals is required every approximately half a year. There may be obvious swelling in local areas after injection, but the swelling can be quickly reduced by cold compress with ice packs.
2. Botulinum toxin: Botulinum toxin has significant advantages in the cosmetic and medical fields, such as safety, being fast, and remarkable effects. However, it also has some shortcomings, such as limited duration of effect, side effects, unnatural facial expressions, and other problems.
3. Regenerative injectables: Regenerative injectables have gained popularity in the field of medical aesthetic filling due to their characteristics of natural quality, long-lasting effects, and safety. However, the widespread application thereof has also been limited due to high costs and technical requirements. With technological advancements and increasing market acceptance, regenerative injectables are expected to become mainstream anti-aging products in the future.
4. Collagen: At present, common collagen injectables on the market include bovine collagen and porcine collagen, which can be absorbed or degraded in the human body and have remarkable repair, regeneration, and whitening effects in the field of medicine and medical aesthetic therapies. However, since bovine/porcine collagen is a heterogeneous protein, the application thereof is only permitted in patients who have passed skin allergy tests.

In summary, after the injection of injectable materials on the market at present, with the gradual degradation of the injected materials, the filling effect also gradually dissipates, and the filling effect is poor in durability, necessitating repeated periodic injections. This approach addresses symptoms rather than the root cause.

### SUMMARY

The technical problem to be solved by the present disclosure is to provide a recombinant collagen filler, a preparation method therefor, and the use thereof, aiming at the problems of poor filling durability, induration in tissues after use, and Tyndall effect in existing filling materials.

To achieve the above objective, in one aspect, the present disclosure provides a recombinant collagen filler, wherein the components of the recombinant collagen filler comprise a recombinant collagen gel and coral hydroxyapatite particles, and the mass ratio of the recombinant collagen gel to the coral hydroxyapatite particles is (2.1-11) : 1.

The filler further optionally comprises a first diluent, and the content of the first diluent is 32.5-45.5% based on the total mass of the recombinant collagen filler being 100%;
the coral hydroxyapatite particles are uniformly distributed in the recombinant collagen gel; and
the recombinant collagen gel is formulated from a recombinant collagen particle and a second diluent.

According to a specific embodiment of the present disclosure, preferably, the content of the first diluent is 35-45% based on the total mass of the recombinant collagen filler being 100%.

According to a specific embodiment of the present disclosure, preferably, the mass ratio of the recombinant collagen gel to the coral hydroxyapatite particles is (4-10) : 1.

The recombinant collagen filler provided by the present disclosure is an injectable medical aesthetic and plastic surgery material. After filling, the recombinant collagen filler can be durable and have a relatively high mechanical strength, and after injection, the recombinant collagen filler can achieve a more natural effect, avoids the Tyndall effect around eyes, and ensures soft tissue texture without induration.

In a frequency sweep test using a rheometer at 37°C and 1% strain at 0.1-100 Hz, G' (storage modulus) is consistently greater than G" (loss modulus), indicating dominant elastic component, and this material is thus defined as a gel. G' represents the elastic component, i.e., the stored portion in the deformation capacity; and G" represents the viscous component, i.e., the lost portion in deformation capacity. Where G" < G', the elastic component is dominant, and the material is thus defined as a gel.

The recombinant collagen filler provided by the present disclosure has a uniform appearance, no layering, no precipitation, and no suspension, and the coral hydroxyapatite particles are uniformly distributed in the gel and uniformly wrapped by the gel. When placed in a container, the filler does not flow when it is either tilted or inverted. When the filler is placed in the container and tilted by 30°-360°, the filler body does not flow along the wall of a bottle or slowly flows down and is in a non-flowing state. According to a specific embodiment of the present disclosure, preferably, the pushing force for the recombinant collagen filler to pass through a 27G needle is 25-35 N, more preferably 28-32 N, further preferably 31 N.

In the present disclosure, the conditions for measuring the pushing force are filling with 2 mL of a recombinant collagen filler sample, fitting a 27G needle, pushing at a constant speed of 10 mm/min until the sample is completely pushed out with a constant force value, thus completing the experiment. During the experiment process, the maximum force value is determined as the pushing force.

According to a specific embodiment of the present disclosure, preferably, the pushing force for the recombinant collagen filler to pass through a 27G needle is about 31 N.

According to a specific embodiment of the present disclosure, preferably, the recombinant collagen filler is subjected to a frequency sweep test at 37°C and 1% strain at 0.1-100 Hz, wherein G' (storage modulus) is consistently greater than G" (loss modulus), indicating dominant elastic component, and this filler is thus a gel.

In the present disclosure, a rheometer is used to characterize the storage modulus G' and the loss modulus G. G' represents the elastic component, i.e., the stored portion in the deformation capacity; and G" represents the viscous component, i.e., the lost portion in deformation capacity. Where G" < G', the elastic component is dominant, and the material is thus a gel; and where G" > G', the viscous component is dominant, and the material is thus a sol.

According to a specific embodiment of the present disclosure, the recombinant collagen gel is obtained by physically cross-linking recombinant collagen microspheres to obtain physically cross-linked recombinant collagen microspheres, adding the physically cross-linked recombinant collagen microspheres to a second diluent, and stirring and swelling the mixture.

According to a specific embodiment of the present disclosure, the recombinant collagen microspheres are formed by subjecting a recombinant human-derived collagen solution to spray granulation.

According to a specific embodiment of the present disclosure, preferably, in the spray granulation step, the spray temperature is 100-260°C, the feed rate is 200-600 ml/h, and the nozzle diameter is 0.75-2 mm.

According to a specific embodiment of the present disclosure, preferably, the mass fraction of a recombinant human-derived collagen in the recombinant human-derived collagen solution is 10%-20%.

The recombinant human-derived collagen described in the present disclosure is a recombinant human-derived collagen disclosed in CN108070032B [METHOD FOR PURIFYING RECOMBINANT HUMAN-DERIVED COLLAGEN].

According to a specific embodiment of the present disclosure, the recombinant human-derived collagen has an amino acid sequence as set forth in SEQ ID No: 1.

According to a specific embodiment of the present disclosure, preferably, the cross-linking mode of the physical cross-linking is thermal cross-linking.

According to a specific embodiment of the present disclosure, preferably, the thermal cross-linking is carried out at a cross-linking temperature of 120-180°C for a cross-linking time of 1-6 h.

According to a specific embodiment of the present disclosure, preferably, the concentration of the recombinant collagen microspheres in the second diluent is 8%-30% (w/v), more preferably 10%-30% (w/v).

According to a specific embodiment of the present disclosure, preferably, the stirring is carried out at a rotating speed of 100-150 r/min for a stirring time of 1-4 h.

According to a specific embodiment of the present disclosure, preferably, the swelling is carried out at a temperature of 2-8°C for a swelling time of 16-24 h.

The recombinant collagen gel described in the present disclosure is obtained by subjecting recombinant collagen microspheres to physical cross-linking and swelling, and the recombinant collagen gel is a uniform gel having no layering, no precipitation, and no suspended particle. In the present disclosure, the quality of the recombinant collagen gel is controlled by controlling the stirring time and the swelling time. If the stirring time is too short and the swelling time is too short, the dissolution will not be well achieved, and if the stirring time is too long and the swelling time is too long, the risk of exceeding the microbial limit will increase. Therefore, in the present disclosure, the recombinant collagen gel obtained by stirring for 1-4 h at a rotating speed of 100-150 r/min and then swelling at 2-8°C for 16-24 h is a mashed-potato-like gel, which does not flow when placed in a container, whether tilted or inverted, is visually uniform, and has no phenomena such as layering, precipitation, and suspension.

According to a specific embodiment of the present disclosure, preferably, the first diluent comprises one of or a combination of two or more of water for injection, purified water, and a phosphate buffer.

According to a specific embodiment of the present disclosure, preferably, the second diluent comprises one of or a combination of two or more of water for injection, purified water, and a phosphate buffer.

According to a specific embodiment of the present disclosure, preferably, the water for injection includes sterile normal saline.

According to a specific embodiment of the present disclosure, preferably, the particle size of the recombinant collagen microspheres is 2-10 µm.

According to a specific embodiment of the present disclosure, preferably, the molecular weight of the recombinant collagen microspheres is 30-90 kDa.

According to a specific embodiment of the present disclosure, preferably, the coral hydroxyapatite particles are prepared by subjecting a coral raw material to a pretreatment, crushing granulation, and hydrothermal exchange.

According to a specific embodiment of the present disclosure, preferably, the pretreatment step comprises subjecting the coral raw material to ultrasonic cleaning to wash impurities, dust, etc. off the surface, and soaking the resulting material in a sodium hypochlorite solution or a hydrogen peroxide solution, followed by cleaning with purified water and drying for later use.

By means of hydrothermal exchange, a mineral ingredient in natural coral stone is converted into natural coral hydroxyapatite, which has a "nano-flower" structure. The natural coral hydroxyapatite particles obtained above are further sieved to obtain coral hydroxyapatite particles meeting the requirements of the present disclosure.

According to a specific embodiment of the present disclosure, preferably, the particle size distribution range of the coral hydroxyapatite particles is 20-50 µm.

According to a specific embodiment of the present disclosure, preferably, the porosity of the coral hydroxyapatite particles is 50-70%.

According to a specific embodiment of the present disclosure, preferably, the conversion rate of the coral hydroxyapatite particles (the content of hydroxyapatite) is ≥ 80%.

According to a specific embodiment of the present disclosure, the raw material of the coral hydroxyapatite particles includes natural coral.

According to a specific embodiment of the present disclosure, preferably, the natural coral includes *Porites* and/or *Goniopora,* more preferably *Porites.*

As for the injectable recombinant collagen filler gel described in the present disclosure, the natural coral hydroxyapatite particles having a "nano-flower" structure are closely combined with the recombinant collagen gel formed by swelling the physically cross-linked recombinant collagen microspheres to obtain an injectable recombinant collagen filler. The recombinant collagen gel is safe, non-toxic, biodegradable, excellent in biocompatibility, and free of viral hazards. The natural coral hydroxyapatite particles have a porosity of 50%-70%, can allow for the loading and incorporation of growth factors, has a controllable degradation time, and maintains a satisfactory degradation performance.

As for the injectable recombinant collagen filler provided by the present disclosure, the recombinant collagen microspheres in the material are 2-10 µm recombinant collagen microspheres formed by spray-drying the recombinant human-derived collagen solution. The above recombinant collagen microspheres are physically cross-linked at a physical cross-linking temperature of 120-180°C for 1-6 h.

The physically cross-linked recombinant collagen microspheres with a particle size of 2-10 µm used in the present disclosure have the advantages below.

The recombinant collagen microspheres obtained by this method are milky white powder, which is safe, non-toxic, biodegradable, excellent in biocompatibility, and free of viral hazards. Compared with a collagen powder obtained by crushing a collagen sponge, the recombinant collagen microspheres have a smaller particle size and more uniform swelling, which is beneficial to injection. The gel formed from the recombinant collagen microspheres can uniformly wrap the natural coral hydroxyapatite particles having a "nano-flower" structure, thereby avoiding the agglomeration of the natural coral hydroxyapatite particles.

In the present disclosure, the mass ratio of the physically cross-linked recombinant collagen gel to the natural coral hydroxyapatite particles is (2.1-11) : 1. In the material prepared by using this mass ratio, the gel formed by physically cross-linking the recombinant collagen microspheres can uniformly wrap the natural coral hydroxyapatite particles having a "nano-flower" structure, thereby avoiding the agglomeration of the natural coral hydroxyapatite particles. Even if it is used after being stored for 24 months, the injection effect can still be maintained. In addition, if the amount of the recombinant collagen microspheres is too large and the amount of the natural coral hydroxyapatite particles is too small, it may result in a low supporting capacity and an excessively fast degradation time; in addition, it is either ineffective or insufficient in promptly stimulating the formation of adequate fibrous connective tissue. If the amount of the recombinant collagen microspheres is too small and the amount of the natural coral hydroxyapatite particles is too large, needle blockage may easily occur during injection; in addition, the mechanical properties are too strong, resulting in an excessively long degradation time in the later stage, and the initial supporting action time may be too short, so it may take an excessively short time to maintain the initial supporting function, which does not allow a sufficient time to stimulate the proliferation of adequate human fibrous connective tissue to replace its supporting function, resulting in collapse at the injection site.

The natural coral hydroxyapatite particles with a particle size of 20-50 µm used in the present disclosure have the advantages below.

When used in an injectable plastic surgery material, hydroxyapatite is often crushed into a powder, and generally the finer the particle size, the better, so that the powder can easily enter human tissues upon injection. The natural coral hydroxyapatite particles in the present disclosure have a particle size of 20-50 µm and a "nano-flower" surface microstructure, can well function to support the skin, has a porosity of 50%-70%, can allow for the loading and incorporation of growth factors, and offers a good degradation time. The recombinant collagen filler obtained by wrapping the natural coral hydroxyapatite particles having a "nano-flower" structure with the recombinant collagen gel of the present disclosure has a good supporting effect and a high elastic modulus, and the most natural effect can be achieved after injection. The tissue at the injection site is soft without induration. The particle size can not only ensure that the filler can be easily injected subcutaneously by a fine needle, but can also ensure that the particle can remain in the treatment site for a long time.

In another aspect, the present disclosure further provides a method for preparing the above recombinant collagen filler, comprising:
(1) mixing coral hydroxyapatite particles with a first diluent under stirring to obtain a coral hydroxyapatite particle solution; and
(2) mixing a recombinant collagen gel with the coral hydroxyapatite particle solution under stirring and subjecting the mixture to swelling and sterilization to obtain the recombinant collagen filler.

In the above preparation method, preferably, the concentration of the coral hydroxyapatite particles in the first diluent is 10%-40% (w/v), preferably 16%-24%.

In the above preparation method, preferably, in step (1), the stirring is carried out at a rotating speed of 100-150 r/min for a stirring time of 30-60 min to obtain a white coral hydroxyapatite particle solution.

In the above preparation method, preferably, in step (2), the stirring is carried out at a rotating speed of 100-150 r/min for a stirring time of 1-4 h.

In the above preparation method, preferably, in step (2), the swelling is carried out at a temperature of 2-8°C for a swelling time of 16-24 h.

In the above preparation method, preferably, in step (2), the ratio of the recombinant collagen gel to the coral hydroxyapatite particles is (2.1-11) : 1.

In order to ensure that the material can be used for human injection, it is very important to ensure sterile conditions during the preparation process. In the present disclosure, the above recombinant collagen gel is mixed with the coral hydroxyapatite particle solution, and the mixture is subjected to moist heat sterilization or irradiation sterilization to obtain the recombinant collagen filler.

In the above preparation method, preferably, in step (2), the sterilization is moist heat sterilization and/or irradiation sterilization; more preferably, the moist heat sterilization is carried out at a temperature of 121-124°C for 15-30 min; and the effective dose for the irradiation sterilization is 15-35 kGy.

In addition, during the specific preparation process, it is also necessary to sterilize all the apparatuses and utensils used. Parts in the apparatuses used that will come into contact with the product, and the utensils used such as scissors and tweezers, are separately wrapped, affixed with moist heat sterilization indicator stickers, and placed in a steam sterilizer for moist heat sterilization at 121°C for 30 min.

In the above preparation method, only how to prepare the injectable recombinant collagen filler provided by the present disclosure is explained. If this material is packaged into a finished product with a syringe, the subsequent packaging steps below are specifically included.

### Filling step:

A filling machine is assembled on an ultra-clean bench. Sterile operation is ensured, and gloves are replaced in time. The prepared material is transferred to the filling machine, and a lower discharge port is opened. The filling machine is adjusted to reach a filling amount of 1.0-1.5 ml.

A disposable syringe is unpackaged in an ultra-clean operating bench and connected to a screwed port at the outlet of the filling machine. A main switch of an air compressor (outside a clean workshop) is turned on, and a filling switch of the filling machine is stepped on to fill the syringe with the material.

### Inner packaging step:

After the filling is complete, a screw cap is screwed on an ultra-clean workbench and then put into a blister shell, which is sealed on a medical heat sealing machine, thus completing the inner packaging.

In another aspect, the present disclosure further provides the above recombinant collagen filler or the above preparation method for use in the preparation of a product for repairing a skin defect.

In some specific embodiments, preferably, the product includes a product for wrinkle correction and/or tissue filling.

The above technical solution of the present disclosure has the advantages below.

The first advantage is that the recombinant collagen gel formed by physically cross-linking the recombinant collagen microspheres wraps the coral hydroxyapatite having a "nano-flower" structure. The color is milky white and opaque, and a Tyndall effect around eyes is avoided after injection. The recombinant collagen gel uniformly wraps the natural coral hydroxyapatite particles to obtain the recombinant collagen filler, the particle size of which is suitable for easy subcutaneous injection via a needle, and the effect can be shown immediately after injection. With the degradation of collagen, the skin itself is stimulated to produce collagen, and the degradation product can provide a raw material for the production of skin collagen. Subsequently, the newly generated collagen occupies the injection site and releases coral hydroxyapatite. The continuous stimulation makes the skin produce fibrous connective tissue, thus achieving the purpose of repairing the skin defect, and the action time is long, leading to no need for multiple injections.

The second advantage is a long-acting effect. The wrinkle removal effect is durable, and for most people, only 1-2 time injections are required.

The third advantage is being fast. The injection process is convenient, and normal activities can be resumed only 3-5 minutes after injection.

The fourth advantage is safety. Collagen is necessary for the human body and can be absorbed by the human body to achieve the purpose of wrinkle removal.

### BRIEF DESCRIPTION OF THE DRAWINGS

FIG. 1 shows scanning electron microscope images of a recombinant collagen gel prepared in Example 1 (in which panel a is 200x magnification and panel b is 1000x magnification).
FIG. 2 shows scanning electron microscope images of 20-50 µm natural coral hydroxyapatite (in which panel a is 100x magnification and panel b is 10000x magnification).
FIG. 3 shows scanning electron microscope images of the recombinant collagen filler prepared in Example 1 (in which panel a is 1000x magnification and panel b is 5000x magnification).
FIG. 4 shows appearance test result images of the fluidity of the recombinant collagen filler prepared in Example 1 (in which panel a: tilted; and panel b: inverted).
FIG. 5 shows a test result diagram of the rheological mechanics (viscoelasticity) of the recombinant collagen filler prepared in Example 1.
FIG. 6 shows the pushing force test result of the recombinant collagen filler prepared in Example 1.
FIG. 7 shows the cell migration results of the recombinant collagen fillers of Examples 1-3.
FIG. 8 shows a hematoxylin-eosin staining picture of the recombinant collagen filler prepared in Example 1 at 52 weeks after implantation in a subcutaneous tissue.

### DETAILED DESCRIPTION

In order to understand the technical features, objectives and beneficial effects of the present disclosure more clearly, the technical solutions of the present disclosure are described in detail as below, but cannot be construed as limitations on the implementable scope of the present disclosure.

During actual production, those skilled in the art can all understand that some related process steps can be adjusted or increased or decreased on the premise that a qualified product can be obtained.

In order to make the objectives, technical solutions, and advantages of the embodiments of the present disclosure clearer, the technical solutions in the embodiments of the present disclosure will be described below clearly and completely with reference to the accompanying drawings in the embodiments of the present disclosure. Apparently, the described embodiments are only some, rather than all, of the embodiments of the present disclosure. All other embodiments that can be obtained by those of ordinary skill in the art without involving creative efforts, based on the embodiments of the present disclosure, shall fall within the scope of protection of the present disclosure.

The amino acid sequence of the recombinant human-derived collagen used in the following examples is:

GPPGEPGNPGKPGSPGPAGSNGEPGPAGSPGEKGSQGSNGNPGPAGNQGQ
PGNKGSPGNPGKPGEPGSNGPQGEPGSQGNPGKNGQPGSPGSQGSPGNQGQPGKPGQP
GEQGSPGNQGPAGNEGPKGQPGQNGKPGSPGPPGEPGNPGKPGSPGPAGSNGEPGPAGS
PGEKGSQGSNGNPGPAGNQGQPGNKGSPGNPGKPGEPGSNGPQGEPGSQGNPGKNGQP
GSPGSQGSPGNQGQPGKPGQPGEQGSPGNQGPAGNEGPKGQPGQNGKPGTPGPPGEPG
NPGKPGSPGPAGSNGEPGPAGSPGEKGSQGSNGNPGPAGNQGQPGNKGSPGNPGKPGEP
GSNGPQGEPGSQGNPGKNGQPGSPGSQGSPGNQGQPGKPGQPGEQGSPGNQGPAGNEG
PKGQPGQNGKP (SEQ ID No: 1).

### Example 1

1. A method for preparing a recombinant collagen gel used for a recombinant collagen filler, comprising:
   Step 1: a recombinant human-derived collagen (a protein having an amino acid sequence as set forth in SEQ ID No: 1 in the sequence listing) and sterile normal saline were formulated into a 15% (wt) recombinant human-derived collagen solution;
   Step 2: the recombinant human-derived collagen solution prepared in step 1 above was subjected to spray granulation on a spray dryer, and the sprayed recombinant collagen microspheres in a collection bottle of the spray dryer was observed as white powder, wherein the particle size of the recombinant collagen microspheres was 2-10 µm;
   Step 3: in the above spray-granulation preparation method for the recombinant collagen microspheres, the spray temperature was 140°C, the feed rate was 300 ml/h, and the nozzle diameter was 0.75 mm;
   Step 4: the recombinant collagen microspheres in step 2 above were physically cross-linked at a cross-linking temperature of 170°C for 2 h; and
   Step 5: the physically cross-linked recombinant collagen microspheres in step 4 above were mixed with sterile normal saline and then stirred at a rotating speed of 100 r/min for 1 h, followed by swelling in a refrigerator at 2-8°C for 16 h, to obtain the recombinant collagen gel, wherein the concentration of the recombinant human-derived collagen microspheres in the normal saline was 16% (w/v), i.e., recombinant human-derived collagen microspheres / (recombinant human-derived collagen microspheres + normal saline) × 100% = 16%.
2. A method for preparing 20-50 µm natural coral hydroxyapatite microspheres used for a recombinant collagen filler, comprising:
   Step 1: natural high-quality *Porites* stone was subjected to ultrasonic cleaning to wash impurities, dust, etc. off the surface, and then soaked in a 5% sodium hypochlorite solution, followed by washing with purified water and drying for later use;
   Step 2: by means of a "hydrothermal exchange" method, the mineral ingredient in the natural coral stone was converted into natural coral hydroxyapatite;
   Step 3: the above processed natural coral hydroxyapatite was sieved to obtain coral hydroxyapatite particles having a particle size range of 20-50 µm, and the porosity and conversion rate thereof are as shown in Table 2; and
   Step 4: the natural coral hydroxyapatite particles were uniformly mixed with sterile normal saline and stirred at a rotating speed of 100 r/min for 30 min to obtain a natural coral hydroxyapatite particle solution at a concentration of 16% (w/v), i.e., natural coral hydroxyapatite particles / (natural coral hydroxyapatite particles + normal saline) × 100% = 16%.
3. A method for preparing the recombinant collagen filler, comprising:
   mixing the above recombinant collagen gel with the natural coral hydroxyapatite particle solution, wherein the mass ratio of the recombinant collagen gel to the natural coral hydroxyapatite particles was 25 : 4, and then stirring the mixture at a rotating speed of 100 r/min for 1 h, followed by swelling in a refrigerator at 2-8°C for 16 h, and then performing moist heat sterilization at 121°C for 30 min to obtain the recombinant collagen filler.

### Example 2

The conditions were the same as in Example 1, except that
1. a recombinant human-derived collagen (a protein having an amino acid sequence as set forth in SEQ ID No: 1 in the sequence listing) was dissolved in water to formulate a 12% (wt) recombinant human-derived collagen solution;
2. during the preparation method for the recombinant collagen microspheres by spray granulation, the spray temperature was 170°C, the feed rate was 600 ml/h, and the nozzle diameter was 1.5 mm; and the particle size of the recombinant collagen microspheres was 2-10 µm; and
3. the recombinant collagen microspheres were physically cross-linked at a cross-linking temperature of 170°C for 3 h.

### Example 3

The conditions were the same as in Example 1, except that
1. the natural coral hydroxyapatite particles were uniformly mixed with sterile normal saline and stirred at a rotating speed of 100 r/min for 30 min to obtain a natural hydroxyapatite particle solution, wherein the concentration of the natural coral hydroxyapatite particles in the normal saline was 24% (w/v); and
2. the mass ratio of the above recombinant collagen gel to the natural coral hydroxyapatite particles was 4 : 1.

### Comparative Example 1

The conditions were the same as in Example 1, except that 1. nano-scaled hydroxyapatite (CAS No.: 719892-61-2) available on the market was purchased; 2. the concentration of the nano-scaled hydroxyapatite in the normal saline was 24% (w/v); and 3. the mass ratio of the recombinant collagen gel to the nano-scaled hydroxyapatite particles was 4 : 1.

### Comparative Example 2

The conditions were the same as in Example 1, except that 1. the recombinant human-derived collagen solution was subjected to freeze drying, instead of spray granulation, to form a recombinant collagen sponge, and the recombinant collagen sponge was then physically cross-linked; and 2. the recombinant collagen sponge was crushed into a powder by a crusher in which the rotating speed of the crusher was 5000 rpm and the time was set to 5 s, and the obtained recombinant collagen powder was mixed with sterile normal saline to obtain a recombinant collagen gel.

### Comparative Example 3

The conditions were the same as in Example 1, except that 1. after spray granulation, the recombinant collagen microspheres were not physically cross-linked; and 2. after spray granulation, the recombinant collagen microspheres were mixed with sterile normal saline to obtain a recombinant collagen gel.

### Comparative Example 4

The conditions were the same as in Example 1, except that 1. the particle size range of the natural coral hydroxyapatite particles was 80-250 µm.

### Comparative Example 5

The conditions were the same as in Example 1, except that 1. the concentration of the recombinant collagen microspheres in the normal saline was 8% (w/v); 2. the concentration of the natural coral hydroxyapatite particles in the normal saline was 43% (w/v); and 3. the mass ratio of the recombinant collagen gel to the nano-scaled hydroxyapatite particles was 3 : 2.

### Comparative Example 6

The conditions were the same as in Example 1, except that 1. the concentration of the recombinant collagen microspheres in the normal saline was 50% (w/v); 2. the concentration of the natural coral hydroxyapatite particles in the normal saline was 10% (w/v); and 3. the mass ratio of the recombinant collagen gel to the nano-scaled hydroxyapatite particles was 12:1.

### Effect Examples

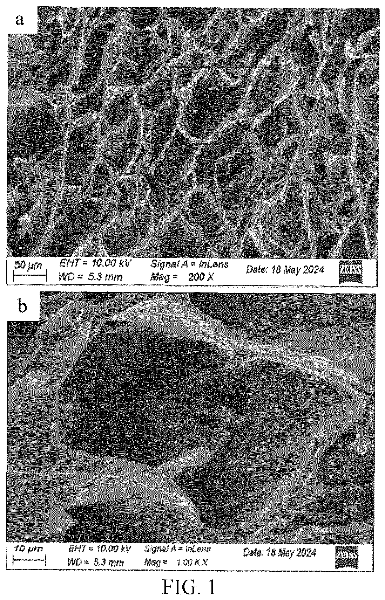

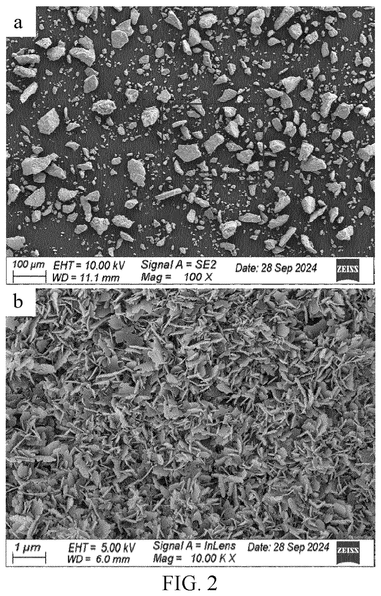

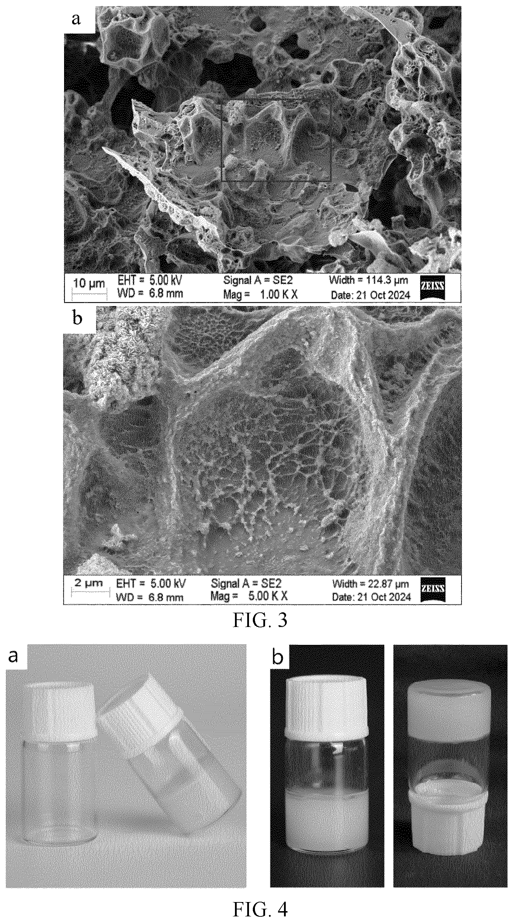

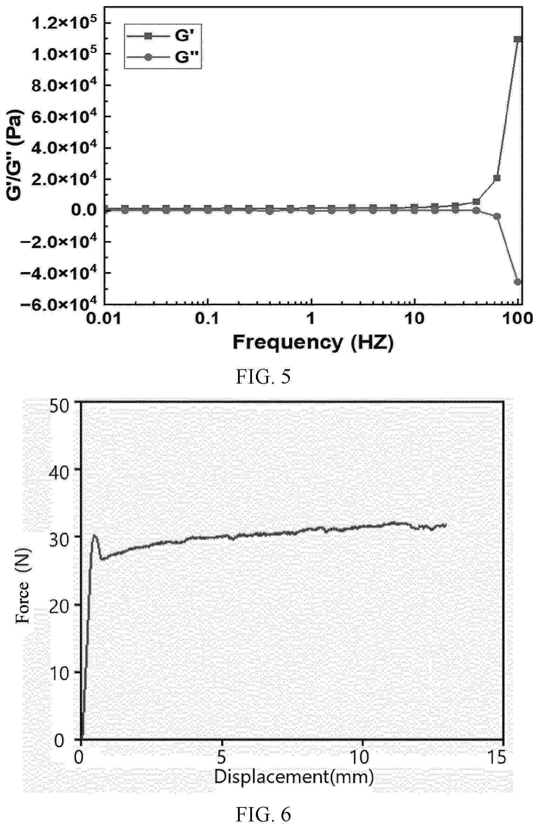

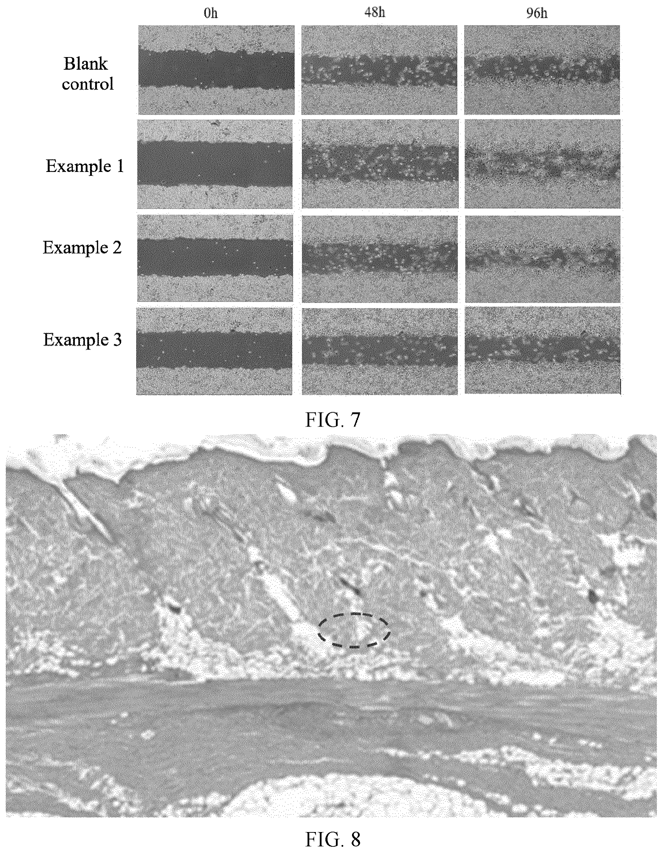

The following performance tests were carried out on the recombinant collagen fillers prepared in the above examples and comparative examples:
1. Appearance test method: 2 mL of the materials prepared in Examples 1, 2, and 3, and Comparative Examples 1, 2, 3, 4, 5, and 6 were randomly taken and pre-filled in syringes. Whether there were unswollen collagen particles and hydroxyapatite particles in the materials was observed. The results are shown in Table 1.
2. Scanning electron microscopy: The recombinant collagen gel and the recombinant collagen filler material in Example 1 were freeze-dried, then put into liquid nitrogen for 3-5 min, and then separated apart, and the cross section thereof and the natural coral hydroxyapatite particles were observed for micromorphology by a scanning electron microscope. The results are as shown in FIGs. 1, 2, and 3.
3. Microbial limit: The materials in Examples 1, 2, and 3, and Comparative Examples 1, 2, 3, 4, 5, and 6 were randomly taken and measured according to Microbial Limit Examination of Non-sterile Products: Microbial Enumeration Method, Chinese Pharmacopoeia, 2020 Edition, Volume IV (General Rule 1105). The results are shown in Table 1.
4. Fluidity:
   The materials in Examples 1, 2, and 3, and Comparative Examples 1, 2, 3, 4, 5, and 6 were randomly taken, pre-filled in syringes, and injected into transparent and colorless glass bottles, which were tilted by 30°-360° to observe whether the material body flowed along the wall of the bottle or flowed down slowly. The results are shown in Table 2 and FIG. 4 (in which panel a: tilted; panel b: inverted).
5. Rheological mechanics:
   Using a conical plate with a diameter of 20 mm and an angle of 1°, the diameter and thickness of the sample in Example 1 were adjusted to 20 mm and 37 µm. An oscillation mode was selected. A frequency sweep test was carried out at 37°C and 1% strain at a frequency of 0.1-100 Hz. A rheometer was used to characterize the storage modulus G' and the loss modulus G. G' represents the elastic component, i.e., the stored portion in the deformation capacity; and G" represents the viscous component, i.e., the lost portion in deformation capacity. Where G" < G', the elastic component is dominant, and the material is thus a gel; and where G" > G', the viscous component is dominant, and the material is thus a sol. The results are as shown in FIG. 5.
6. The pushing force was tested on a universal mechanical testing machine. 2 mL of the sample of Example 1 after filling was fitted with a 27G needle, fixed on a mechanical testing machine, and pushed at a constant speed of 10 mm/min until the sample was pushed out with a constant force value, thus completing the experiment. A curve of the pushing force of the sample over time during pushing was recorded. The results are shown in Table 2 and FIG. 6.
7. Biological evaluation:
   Test according to GB/T 16886: The samples prepared in Examples 1, 2, and 3, and Comparative Examples 1, 2, 3, 4, 5, and 6 were subjected to a cytotoxicity experiment, and Examples 1, 2, and 3 were subjected to a cell migration experiment. The results are shown in Tables 3 and 4 and FIG. 7.
8. Porosity test:
   The materials in Examples 1, 2, and 3, and Comparative Examples 1, 2, 3, 4, 5, and 6 were freeze-dried, then sputter-coated with gold, and subjected to a scanning electron microscope test. The results are as shown in Table 2.
9. Conversion rate:
   The samples prepared in Examples 1, 2, and 3, and Comparative Examples 1, 2, 3, 4, 5, and 6 were measured by a thermogravimetric analyzer for the proportion of the natural coral mineral ingredient converted into hydroxyapatite. The results are as shown in Table 2.

**Table 1 Appearance test and microbial limit results**

| Example | Appearance | Microbial limit |
|---|---|---|
| Example 1 | Uniform, no layering, no precipitation, and no suspended particle | Total number of aerobic bacteria: 1 cfu/ml |
| | | Total number of molds and yeasts: 0 cfu/ml |
| Example 2 | Uniform, no layering, no precipitation, and no suspended particle | Total number of aerobic bacteria: 2 cfu/ml |
| | | Total number of molds and yeasts: 0 cfu/ml |
| Example 3 | Uniform, no layering, no precipitation, and no suspended particle | Total number of aerobic bacteria: 60 cfu/ml |
| | | Total number of molds and yeasts: 5 cfu/ml |
| Comparative Example 1 | Uniform, no layering, no precipitation, and no suspended particle | Total number of aerobic bacteria: 2 cfu/ml |
| | | Total number of molds and yeasts: 0 cfu/ml |
| Comparative Example 2 | Non-uniform, layered, no precipitation, with suspended collagen particles | Total number of aerobic bacteria: 20 cfu/ml |
| | | Total number of molds and yeasts: 3 cfu/ml |
| Comparative Example 3 | Uniform, layered, with precipitation and suspended particles | Total number of aerobic bacteria: 18 cfu/ml |
| | | Total number of molds and yeasts: 1 cfu/ml |
| Comparative Example 4 | Uniform, layered, no precipitation, and no suspended particle | Total number of aerobic bacteria: 5 cfu/ml |
| | | Total number of molds and yeasts: 0 cfu/ml |
| Comparative Example 5 | Uniform, layered, with precipitation and suspended particles | Total number of aerobic bacteria: 3 cfu/ml |
| | | Total number of molds and yeasts: 1 cfu/ml |
| Comparative Example 6 | Uniform, layered, no precipitation, and no suspended particle | Total number of aerobic bacteria: 2 cfu/ml |
| | | Total number of molds and yeasts: 0 cfu/ml |

As can be seen from the above table, by comparing Example 1 with Example 3, it is found that the stirring time and the swelling time of the prepared material were the maximum values of the parameters. Although the microbial limit results are qualified, the risk of the microbial limit exceeding the standard is increased. Therefore, it is more appropriate to maintain the stirring time and swelling time not more than the maximum values during the preparation of the material.

As can be seen from the above table, by comparing Example 1 with Comparative Example 2, it is found that the sample prepared by Comparative Example 2 is non-uniform and layered and has suspended collagen particles. After analysis, the reason lies in that the freeze-dried recombinant collagen sponge was crushed by a crusher, resulting in the particle size of the collagen being not fine; and during the compounding process with hydroxyapatite, its swelling effect is inferior to that of the recombinant collagen powder prepared by spray granulation, which has finer particles.

As can be seen from the above table, by comparing Example 1 with Comparative Example 3, it is found that the sample prepared in Comparative Example 3 is uniform and layered and has precipitation and suspended particles. After analysis, the reason lies in that the recombinant collagen gel formed from the recombinant collagen microspheres without physical cross-linking has a low viscoelasticity, which is insufficient to uniformly disperse the natural coral hydroxyapatite, precipitation occurs due to the action of gravity, and there are suspended particles, leading to the aggregation of the natural coral hydroxyapatite particles.

As can be seen from the above table, by comparing Example 1 with Comparative Example 5, it is found that the sample prepared in Comparative Example 5 is uniform and layered and has precipitation and suspended particles. After analysis, the reason lies in that the content of the natural coral hydroxyapatite is too high, so that the gel formed from the recombinant collagen microspheres cannot uniformly wrap the natural coral hydroxyapatite particles having a "nano-flower" structure, leading to the phenomenon of aggregation of the coral hydroxyapatite particles.

**Table 2 Detection results of recombinant collagen fillers**

| | Flow behavior | Porosity | Conversion rate (hydroxyapatite content) | Pushing force |
|---|---|---|---|---|
| Example 1 | Non-flowing | 65% | 80% | 31 N |
| Example 2 | Non-flowing | 55% | 83% | 38 N |
| Example 3 | Non-flowing | 64% | 86% | 28 N |
| Comparative Example 1 | Non-flowing | 30% | 99.9% | 30N |
| Comparative Example 2 | Non-flowing | 55% | 83% | Blockage |
| Comparative Example 3 | Flowing | 64% | 86% | 20 N |
| Comparative Example 4 | Non-flowing | 55% | 80% | Blockage |
| Comparative Example 5 | Non-flowing | 57% | 78% | Blockage |
| Comparative Example 6 | Flowing | 55% | 76% | 10 N |

From the results in the above table, it can be seen that the 20-50 µm natural coral hydroxyapatite has a porosity of 50%-70%, can allow for the loading and incorporation of growth factors, and has a conversion rate of more than 80% and a good degradation time.

From the results in the above table, it can be seen that the nano-scaled hydroxyapatite in Comparative Example 1 has a lower porosity, a comparable pushing force to that of the natural coral hydroxyapatite, a high hydroxyapatite content, and a longer degradation time.

From the results in the above table, it can be seen that as for the crushed collagen powders in Comparative Examples 2, 4, and 5, the coral hydroxyapatite particles have an excessively large particle size and an excessively high content, both of which lead to needle tube blockage, causing inconvenience for clinical operation.

FIG. 5 shows a test result diagram of the rheological mechanics (viscoelasticity) of the recombinant collagen filler in Example 1.

As shown in FIG. 5, when the oscillation frequency is 1 Hz, the recombinant collagen filler has G' of 1493.7 Pa and G" of -111.997 Pa; when the oscillation frequency is increased to 10 Hz, the recombinant collagen filler has G' of 1974.43Pa and G" of 87.7038 Pa; and when the oscillation frequency is increased to 100 Hz, the recombinant collagen filler has G' of 109281 Pa and G" of -45522.4 Pa. The strain sweep results are as shown above. In the strain range of the frequency sweep test at 37°C and 1% strain, the G' (storage modulus) of the recombinant collagen filler is consistently greater than G" (loss modulus), indicating dominant elastic component, and this filler is thus a gel.

FIG. 6 shows the pushing force test result of the recombinant collagen filler in Example 1.

As can be seen from FIG. 6, the pushing force for the recombinant collagen filler to pass through a 27G needle is about 31 N.

The following is the biocompatibility evaluation of the recombinant collagen filler.

**Table 3 Cytotoxicity results**

| | | | | | |
|---|---|---|---|---|---|
| Example | Cell viability | | | | |
| | Blank group | Material | | | |
| Example 1 | | 100% | 50% | 25.00% | 12.50% |
| | 100.00% | 95.40% | 99.77% | 102.94% | 109.49% |
| Example 2 | 100.01% | 95.81% | 102.11% | 108.90% | 112.35% |
| Example 3 | 100.20% | 99.15% | 104.34% | 106.73% | 105.85% |
| Comparative Example 1 | 100.11% | 85.81% | 90.31% | 95.40% | 98.15% |
| Comparative Example 2 | 100.00% | 89.17% | 94.22% | 96.53% | 101.07% |
| Comparative Example 3 | 100.00% | 93.35% | 98.18% | 103.22% | 106.58% |
| Comparative Example 4 | 100.50% | 85.58% | 90.33% | 96.56% | 98.88% |
| Comparative Example 5 | 100.00% | 88.81% | 92.11% | 98.71% | 102.44% |
| Comparative Example 6 | 100.44% | 90.16% | 97.21% | 102.76% | 105.06% |

**Table 4 Cell migration results**

| | Migration rate at 48 h | | Migration rate at 96 h | |
|---|---|---|---|---|
| | Average value | SD | Average value | SD |
| Blank control | 100.00% | 6.62% | 100.00% | 6.17% |
| Example 1 | 101.20% | 9.87% | 116.96% | 7.14% |
| Example 2 | 111.37% | 13.44% | 135.94% | 8.33% |
| Example 3 | 111.09% | 7.23% | 139.36% | 6.50% |

FIG. 7 shows the cell migration results.

### Biological evaluation results:

The cytotoxicity test results are shown in Table 3. The results show that the original leaching solutions of the recombinant collagen fillers acted on L929 cells for 24 h, and the cell viabilities were all not less than 85%, without obvious cytotoxicity.

According to the cell migration results shown in FIG. 7, one dosage concentration was set for each sample, and a cell scratch experiment was carried out on L929 cells. The cell migration rate in the blank control group was used for normalization to calculate the relative migration rate in each group. The test results are shown in Table 4 and FIG. 7. Compared with the blank control group, the materials prepared in Examples 1, 2, and 3 can significantly promote the migration of L929 cells at both 48 h and 96 h.

10. Filling effect test: 1 ml of samples of Examples 1, 2, and 3, and Comparative Examples 1, 2, 3, 4, 5, and 6 after filling were fitted with a 27G needle, and 0.2 ml of the samples were respectively injected into the subcutaneous tissue of the back of SD rats. The appearance at the injection site was observed at 4 weeks, 8 weeks, 12 weeks, 26 weeks, and 52 weeks after implantation, including whether there were redness and swelling, inflammation, the influence of the sample on the surrounding tissues, and the degradation of the material, as shown in Table. 5.

**Table 5 Gross observation of subcutaneous implantation**

| | 4 weeks | 8 weeks | 12 weeks | 26 weeks | 52 weeks |
|---|---|---|---|---|---|
| Example 1 | Soft tissue, without induration, with bulges and smooth epidermis | Soft tissue, without induration, with bulges and smooth epidermis | Soft tissue, without induration, with bulges and smooth epidermis | Soft tissue, without induration, with bulges and smooth epidermis | Soft tissue, without induration, with almost resolved bulges, and smooth epidermis |
| Example 2 | Soft tissue, without induration, with bulges and smooth epidermis | Soft tissue, without induration, with bulges and smooth epidermis | Soft tissue, without induration, with bulges and smooth epidermis | Soft tissue, without induration, with bulges and smooth epidermis | Soft tissue, without induration, with rice-grain-sized bulges and smooth epidermis |
| Example 3 | Soft tissue, without induration, with bulges and smooth epidermis | Soft tissue, without induration, with bulges and smooth epidermis | Soft tissue, without induration, with bulges and smooth epidermis | Soft tissue, without induration, with bulges and smooth epidermis | Soft tissue, without induration, with soybean-sized bulges and smooth epidermis |
| Comparative Example 1 | Inflammation with redness and swelling; | With redness and swelling | Soft tissue, without induration, with bulges and smooth epidermis | No edema in the tissue, with resolved bulges, and smooth epidermis | / |
| Comparative Example 2 | Inflammation with redness and swelling; | With redness and swelling | No edema in the tissue, with bulges and smooth epidermis | No edema in the tissue, with bulges reduced in size and smooth epidermis | Soft tissue, without induration, with almost resolved bulges, and smooth epidermis |
| Comparative Example 3 | Soft tissue, without induration, with bulges and smooth epidermis | Soft tissue, without induration, with bulges and smooth epidermis | Soft tissue, without induration, without bulges, with smooth epidermis | No edema in the tissue, with resolved bulges, and smooth epidermis | / |
| Comparative Example 4 | Inflammation with redness and swelling; | With redness and swelling | Soft tissue, without induration, with bulges and smooth epidermis | No edema in the tissue, with resolved bulges, and smooth epidermis | / |
| Comparative Example 5 | Inflammation with redness and swelling; | With redness and swelling | No edema in the tissue, with bulges and smooth epidermis | No edema in the tissue, with basically no change, with smooth epidermis | No edema in tissue, with bulges slightly bigger than a soybean, with sunken epidermis |
| Comparative Example 6 | Soft tissue, without induration, with bulges and smooth epidermis | Soft tissue, without induration, with bulges and smooth epidermis | No edema in the tissue, with resolved bulges, and smooth epidermis | / | / |

As can be seen from the above table, by comparing Example 1 with Example 2, it is found that an increased physical cross-linking time of the recombinant collagen powder resulting from spray granulation leads to a longer degradation time in vivo. By comparing Example 1 and Example 3, it is found that a high content of the natural coral hydroxyapatite leads to a longer degradation time in vivo.

As can be seen from the above table, by comparing Example 1 with Comparative Examples 1 and 4, it is found that the use of the 20-50 µm natural coral hydroxyapatite is more conducive to the incorporation of bioactive factors and the regeneration of collagen and has a more obvious filling effect in vivo than the nano-scaled hydroxyapatite and the natural coral hydroxyapatite with a large pore size.

As can be seen from the above table, by comparing Example 1 with Comparative Examples 2 and 3, it is found that the material prepared using the recombinant collagen microspheres formed by the spray granulation of the solution formulated using the recombinant human-derived collagen described in the present disclosure (a protein having an amino acid sequence as set forth in SEQ ID No: 1 in the sequence listing) has a higher biosafety and an more excellent biocompatibility than the collagen powder formed by crushing the freeze-dried recombinant collagen sponge, and the recombinant collagen prepared by spray granulation and then physical cross-linking has a good degradation time, which can achieve a satisfactory effect.

As can be seen from the above table, by comparing Example 1 with Comparative Examples 5 and 6, it is found that if the amount of the recombinant collagen microspheres is too large and the amount of the natural coral hydroxyapatite powder is too small, it may result in a low supporting capacity and an excessively fast degradation time, which is insufficient in promptly stimulating the formation of adequate fibrous connective tissue. If the amount of the recombinant collagen microspheres is too small and the amount of the natural coral hydroxyapatite particles is too large, needle blockage may easily occur during injection; in addition, the mechanical properties are too strong, resulting in an excessively long degradation time in the later stage, and the initial supporting action time may be too short, so it may take an excessively short time to maintain the initial supporting function, which does not allow a sufficient time to stimulate the proliferation of adequate human fibrous connective tissue to replace its supporting function, resulting in collapse at the injection site.

FIG. 8 is a hematoxylin-eosin staining picture at 52 weeks after the injection of Example 1 was implanted in the subcutaneous tissue of an SD rat. It can be seen that at 52 weeks after implantation with the injection, there is still a small amount of the material left at the implantation site, indicating that the material can persist in the body for not less than one year.

## Claims

1. A recombinant collagen filler, wherein the components of the recombinant collagen filler comprise a recombinant collagen gel and coral hydroxyapatite particles, and the mass ratio of the recombinant collagen gel to the coral hydroxyapatite particles is (2.1-11) : 1;
the filler further optionally comprises a first diluent, and the content of the first diluent is 32.5-45.5% based on 100% of a total mass of the recombinant collagen filler;
the coral hydroxyapatite particles are uniformly distributed in the recombinant collagen gel; and
the recombinant collagen gel is formulated from recombinant collagen microspheres and a second diluent.

2. The recombinant collagen filler according to claim 1, wherein the recombinant collagen gel is obtained by physically cross-linking recombinant collagen microspheres to obtain physically cross-linked recombinant collagen microspheres, adding the physically cross-linked recombinant collagen microspheres to the second diluent, and stirring the mixture to allow swelling.

3. The recombinant collagen filler according to claim 2, wherein the recombinant collagen microspheres are formed by subjecting a recombinant human-derived collagen solution to spray granulation;
preferably, in the spray granulation, the spray temperature is 100-260°C, the feed rate is 200-600 ml/h, and the nozzle diameter is 0.75-2 mm;
preferably, the mass fraction of the recombinant human-derived collagen in the recombinant human-derived collagen solution is 10%-20%;
preferably, the recombinant human-derived collagen has an amino acid sequence of SEQ ID No: 1.

4. The recombinant collagen filler according to claim 2, wherein the physical cross-linking is carried out by thermal cross-linking;
preferably, the thermal cross-linking is carried out at a cross-linking temperature of 120-180°C for 1-6 h;
preferably, the concentration of the recombinant collagen microspheres in the second diluent is 8%-30% (w/v);
preferably, the stirring is carried out at a rotating speed of 100-150 r/min for 1-4 h;
preferably, the swelling is carried out at a temperature of 2-8°C for 16-24 h.

5. The recombinant collagen filler according to claim 1, wherein the first diluent comprises one of or a combination of two or more of water for injection, purified water, and a phosphate buffer;
preferably, the second diluent comprises one of or a combination of two or more of water for injection, purified water, and a phosphate buffer.

6. The recombinant collagen filler according to claim 2, wherein the particle size of the recombinant collagen microspheres is 2-10 µm;
preferably, the molecular weight of the recombinant collagen microspheres is 30-90 kDa.

7. The recombinant collagen filler according to claim 1, wherein the particle size range of the coral hydroxyapatite particles is 20-50 µm;
preferably, the porosity of the coral hydroxyapatite particles is 50-70%;
preferably, the conversion rate of the coral hydroxyapatite particles is ≥ 80%.

8. A method for preparing the recombinant collagen filler according to any one of claims 1 to 7, comprising:
(1) mixing coral hydroxyapatite particles with a first diluent under stirring to obtain a coral hydroxyapatite particle solution; and
(2) mixing a recombinant collagen gel with the coral hydroxyapatite particle solution under stirring, allowing the mixture to swelling, and performing sterilization to obtain the recombinant collagen filler.

9. The preparation method according to claim 8, wherein the concentration of the coral hydroxyapatite particles in the first diluent is 10%-40% (w/v);
or, in step (1), the stirring is carried out at a rotating speed of 100-150 r/min for 30-60 min;
or, in step (2), the stirring is carried out at a rotating speed of 100-150 r/min for 1-4 h;
or, in step (2), the swelling is carried out at a temperature of 2-8°C for 16-24 h;
or, in step (2), the sterilization is moist heat sterilization and/or irradiation sterilization; more preferably, the moist heat sterilization is carried out at a temperature of 121-124°C for 15-30 min; and the effective dose for the irradiation sterilization is 15-35 kGy.

10. The recombinant collagen filler according to any one of claims 1 to 7 or the preparation method according to claim 8 or 9 for use in the preparation of a product for repairing a skin defect,
preferably, the product includes a product for wrinkle correction and/or tissue filling.
